# EUROPÄISCHE PATENTSCHRIFT

(11) **EP 3 773 351 B1**
(45) Veröffentlichungstag und Bekanntmachung des Hinweises auf die Patenterteilung: **25.08.2021**
(21) Anmeldenummer: 19715444.6
(22) Anmeldetag: 01.04.2019
(51) Int. Cl.: A61F 2/50, A61F 5/01

(54) **ORTHESEN- ODER PROTHESEN-SYSTEM ZUR ORTHESEN- ODER PROTHESENSTEUERUNG ODER -REGELUNG**
ORTHESIS OR PROSTHESIS SYSTEM FOR OPEN-LOOP OR CLOSED-LOOP ORTHESIS OR PROSTHESIS CONTROL
SYSTÈME D'ORTHÈSES OU DE PROTHÈSES POUR LA COMMANDE OU LE RÉGLAGE D'ORTHÈSES OU DE PROTHÈSES

(30) Priorität: 09.04.2018 DE 102018205306
(43) Veröffentlichungstag der Anmeldung: 17.02.2021
(73) Patentinhaber: Ottobock SE & Co. KGaA, 37115 Duderstadt (DE)
(72) Erfinder: RYSCHKA, Martin, 23617 Stockelsdorf (DE); KUSCHE, Roman, 21075 Hamburg (DE)
(74) Vertreter: Gramm, Lins & Partner Patent- und Rechtsanwälte PartGmbB
(86) Internationale Anmeldenummer: PCT/EP2019/058135
(87) Internationale Veröffentlichungsnummer: WO 2019/197189

(56) Entgegenhaltungen:
- WO-A1-2017/160183
- DE-A1-102007 013 660
- US-A- 4 808 187
- US-A1- 2012 004 736

## Beschreibung

Die Erfindung betrifft ein Orthesen- oder Prothesen-System.

Eine Orthese im Sinne der Erfindung ist ein Hilfsmittel, das zur Stabilisierung, Entlastung, Ruhigstellung, Führung oder Korrektur eines Körperteils eingesetzt wird. Eine Prothese im Sinne der Erfindung ist ein Hilfsmittel, welches einen Körperteil ersetzt.

In der Medizin werden Orthesen und Prothesen somit als ergänzende Hilfsmittel bzw. als Ersatz für Gliedmaßen und Organe eingesetzt. Begrifflich sind Orthesen und Prothesen nicht immer trennbar, zumal einige Orthesen auch ausgefallene Körperfunktionen ersetzen. Daher wird im Folgenden stets von "Orthesen oder Prothesen" gesprochen. Es ist selbstverständlich möglich, den Erfindungsgegenstand auch nur bei einer Prothese oder nur bei einer Orthese anzuwenden.

Ausgangspunkt für Überlegungen zur Orthesen- oder Prothesensteuerung oder - regelung ist der Wunsch des Nutzers einer Orthese oder Prothese, diese nicht als Nachteil oder Behinderung zu empfinden, sondern im täglichen Gebrauch im Falle einer Orthese als Unterstützung oder Entlastung des zu kontrollierenden Körperteils bzw. im Falle einer Prothese als möglichst vollständigen Ersatz für den fehlenden Körperteil nutzen zu können.

Das Orthesen- oder Prothesen-System soll somit die Einschränkungen des Nutzers bestmöglich kompensieren bzw. einen Ersatz schaffen, der Einschränkungen verhindert. Anforderungen an ein Orthesen- oder Prothesen-System sind daher beispielsweise die Erhaltung bzw. Wiederherstellung der motorischen Fähigkeiten des Nutzers sowie die Alltagstauglichkeit. Dabei ermöglichen möglichst viele Bewegungs-Freiheitsgrade dem Nutzer eine besonders gute Kompensation von Einschränkungen. Bei einem Orthesen- oder Prothesen-System im Sinne der Erfindung wird die Orthese oder Prothese mit einer Sensorik und einer Aktorik kombiniert, welche gemeinsam in einen Steuerungsablauf oder auch in einen Regelkreis eingefügt sein können.

Unter Sensorik und Aktorik wird dabei im üblichen Sinne die Erfassung von Signalen bzw. die Auslösung von Bewegungen durch Signale verstanden.

Zur Steuerung oder Regelung von Orthesen oder Prothesen muss der Wunsch des Nutzers, eine Bewegung vorzunehmen, möglichst schnell und zuverlässig ermittelt werden. Dies geschieht üblicherweise durch die Erfassung von muskelbezogenen Signalen. Unter muskelbezogenen Signalen werden dabei, insbesondere elektrische, Signale verstanden, die sich auf einen Zustand eines Muskels beziehen, insbesondere auf die Stärke und/oder Richtung der Kontraktion des Muskels oder auf die Richtung und/oder Geschwindigkeit der Bewegung des Muskels.

Von besonderer Wichtigkeit ist dabei die Störsicherheit der erfassten muskelbezogenen Signale, welche für die Regelung verwendet werden, um Fehlfunktionen des Orthesen- oder Prothesen-Systems zu verhindern.

Im Stand der Technik sind verschiedene Verfahren zur Erfassung von muskelbezogenen Signalen bekannt, die zur Steuerung oder Regelung von Orthesen oder Prothesen eingesetzt werden können:
Die DE 10 2008 002 933 A1 zeigt eine Datenaufzeichnung zur Patientenanalyse, wie sie bei herkömmlichen Prothesen genutzt werden kann. Dabei wird an wenigen, zumeist zwei Orten mittels unterschiedlicher Oberflächenelektroden die elektrische Aktivität der Muskeln oder Muskelgruppen, beispielsweise durch Elektromyografie (EMG), erfasst und analysiert.

Eine weitere bekannte Methode zur Detektion von Muskelaktivitäten ist die Mechanomyografie (MMG), bei der die Vibrationen, die bei der Muskelanspannung entstehen, gemessen werden.

Wissenschaftliche Veröffentlichungen zeigen weiterhin, dass auch der Betrag der komplexen elektrischen Bioimpedanz durch Muskelkontraktionen beeinflusst wird (vgl. Soo- Chan Kim: Estimation of Hand Gestures Using EMG and Bioimpedance,The Transactions of the Korean Institute of Electrical Engineers, Vol. 65, No. 1, pp. 194-199, 2016, ISSN 1975-8359 (Print) / ISSN 2287-4364 (Online), http://dx.doi.org/10.5370/KIEE.2016.65.1.194), wobei sich die Diskussion auf die Art und Weise von möglichen Messungen mit Sensoren oder Elektroden und die Interpretation von Messergebnissen beschränkt.

Aus der WO 2017/160183 A1 ist ein Verfahren zur bionischen Steuerung einer technischen Einrichtung, wie beispielsweise eines Exoskeletts oder einer Prothese, bekannt. Mittels Elektroden werden elektrophysiologische Signale eines kontrahierenden Muskels erfasst und einer Steuereinrichtung zugeleitet. Zur Erfassung des elektrophysiologischen Signals wird eine Spannung an den Muskel angelegt und eine Änderung der elektrischen Impedanz bei Muskelkontraktion erfasst und als Signal verwendet.

Der Erfindung liegt die Aufgabe zugrunde, ein Orthesen- oder Prothesen-System bereit zu stellen, welches jeweils eine verbesserte Störsicherheit aufweist. Insbesondere ist es wünschenswert, möglichst gut zwischen Störeinflüssen und tatsächlicher Muskelkontraktion differenzieren zu können.

Diese Aufgabe wird gelöst durch ein Orthesen- oder Prothesen-System gemäß Anspruch 1. Vorteilhafte Ausgestaltungen der Erfindung sind in den Unteransprüchen enthalten.

Das betrachtete Orthesen- oder Prothesen-System weist die folgenden Komponenten auf: wenigstens eine Orthese oder Prothese, wenigstens ein Paar von Elektroden, welche zur Kontaktierung des Körpers des Nutzers der Orthese oder Prothese vorgesehen sind, zur Erfassung muskelbezogener Signale, wenigstens eine Auswerteeinheit für von dem wenigstens einen Elektrodenpaar erfasste muskelbezogene Signale, wenigstens einen Aktor zur Bewegung der wenigstens einen Orthese oder Prothese und wenigstens eine Steuereinheit zur Steuerung des wenigstens einen Aktors.

Gemäß einem ersten Aspekt der Erfindung ist das wenigstens eine Elektrodenpaar dazu eingerichtet, wenigstens ein erstes muskelbezogenes Signal unter Verwendung einer ersten Messfrequenz und ein zweites muskelbezogenes Signal unter Verwendung einer zweiten Messfrequenz zu erfassen. Außerdem ist die wenigstens eine Auswerteeinheit dazu eingerichtet, eine Phase des jeweiligen ersten Signals und eine Phase des jeweiligen zweiten Signals auszuwerten. Die Auswertung der Phase des jeweiligen ersten Signals und der Phase des jeweiligen zweiten Signals kann es ermöglichen, ein "echtes" muskelbezogenes Signal von einem Störsignal zu unterscheiden.

In einer bevorzugten Ausführung der Erfindung ist die jeweilige Auswerteeinheit dazu eingerichtet, einen Phasenverlauf des jeweiligen ersten und jeweiligen zweiten Signals auszuwerten. Wie nachfolgend beschrieben wird, kann der Phasenverlauf eines muskelbezogenen Signals bestimmte Charakteristiken aufweisen, die bei einem Störsignal nicht auftreten. So kann ein "echtes" muskelbezogenes Signal von einem beliebigen andersartigen Signal, hier Störsignal genannt, unterschieden werden.

Die jeweilige Auswerteeinheit ist dazu eingerichtet:
- eine Phase des jeweiligen ersten Signals mit einer, insbesondere frequenzabhängigen, Referenzphase zu vergleichen, um eine erste Phasenveränderung wenigstens qualitativ zu ermitteln;
- eine Phase des jeweiligen zweiten Signals mit der, insbesondere frequenzabhängigen, Referenzphase zu vergleichen, um eine zweite Phasenveränderung wenigstens qualitativ zu ermitteln; und
- die erste Phasenveränderung und die zweite Phasenveränderung auszuwerten, insbesondere miteinander zu vergleichen.

Dadurch könnend die Charakteristiken eines echten muskelbezogenen Signals bzw. eines Störsignals erkannt und ausgewertet werden.

In einer bevorzugten Ausführung ist die Referenzphase eine, insbesondere zuvor bestimmte, Phase, die im Wesentlichen einer Messung in einem entspannten Zustand eines Muskels entspricht.

Die Verwendung einer solchen Referenzphase kann dazu beitragen, dass die Signale richtig erkannt werden.

Nach einer bevorzugten Ausführung ist die Auswerteeinheit dazu eingerichtet zu ermitteln, ob die erste Phasenveränderung eine Phasenveränderung in die gleiche Richtung wie die zweite Phasenveränderung ist, oder in eine entgegengesetzte Richtung.

Die Richtungen der ersten und zweiten Phasenveränderung können zur Unterscheidung von echten muskelbezogenen Signalen und Störsignalen dienen.

Nach einer Ausführungsform ist die Auswerteeinheit dazu eingerichtet ist, das erste und zweite Signal als zu einer Muskelkontraktion gehörend zu werten, wenn die erste und zweite Phasenveränderung Phasenveränderungen in entgegengesetzte Richtungen sind.

Auf diese Weise kann ein echtes muskelbezogenes Signal zuverlässig erkannt werden.

Entsprechend kann nach einer Ausführung die Auswerteeinheit dazu eingerichtet sein, das erste und zweite Signal als zu einer Störung gehörend zu werten, wenn die erste und zweite Phasenveränderung Phasenveränderungen in die gleiche Richtung sind.

Somit kann eine Störung bzw. ein Störsignal richtig erkannt werden.

Nach einer Ausführung beträgt die erste Messfrequenz mehr als ca. 60 kHz und die zweite Messfrequenz weniger als ca. 60 kHz.

Die Erfinder haben festgestellt, dass sich der Phasenverlauf unterhalb einer Frequenz von ca. 60 kHz von dem Phasenverlauf oberhalb einer Frequenz von ca. 60 kHz unterscheidet, je nachdem, ob das auszuwertende Signal ein echtes muskelbezogenes Signal oder ein Störsignal ist. Bei einer Messung unterhalb und oberhalb von ca. 60 kHz kann also eine zuverlässige Unterscheidung erfolgen.

Nach einer bevorzugten Ausführung ist die erste Messfrequenz wesentlich größer als 60 kHz und / oder die zweite Messfrequenz wesentlich kleiner als 60 kHz. Dadurch kann die Unterscheidung zwischen den zwei Arten von Signalen mit noch größerer Zuverlässigkeit erfolgen.

In einer bevorzugten Ausführung der Erfindung ist das wenigstens erste und zweite muskelbezogene Signal ein (komplexes) Bioimpedanz-Signal.

Insbesondere bei einem Bioimpedanz-Signal tritt das zuvor beschriebene charakteristische Phasenverhalten auf.

In einer bevorzugten Ausführung der Erfindung ist das wenigstens eine Elektrodenpaar weiterhin dazu eingerichtet, dem Nutzer der Orthese oder Prothese elektrische Stimulationssignale zuzuführen. Durch die Nutzung desselben Elektrodenpaares wie für die Signalmessungen wird wiederum der apparative Aufwand gesenkt, und es wird sichergestellt, dass die Stimulationssignale genau am Messort der Signalmessungen zugeführt werden und somit im Bereich desselben Muskels erfolgen, auf den auch die Signalmessungen bezogen sind.

In einer bevorzugten Variante dieser Ausführung sind die elektrischen Stimulationssignale dazu eingerichtet, dem Nutzer der Orthese oder Prothese ein Feedback über die Bewegungen und/oder den Zustand der Orthese oder Prothese zu geben. Bewegungs- oder Zustandsparameter, auf die sich das Feedback beziehen kann, sind insbesondere die Geschwindigkeit der Orthese oder Prothese oder die von dieser erzeugte Kraft. Auf diese Weise kann beispielsweise dem Nutzer einer Handprothese mitgeteilt werden, wie kräftig die Prothese zugreift.

Eine modulare Nachrüstung bestehender Orthesen- oder Prothesen-Systeme ist möglich.

Damit ist das erfindungsgemäße Orthesen- oder Prothesen-System für die unterschiedlichsten Anwendungen in der Medizintechnik einsetzbar.

Insbesondere ermöglicht die Erfindung eine Verbesserung der Störsicherheit der Orthesen- oder Prothesensteuerung durch die Erfassung wenigstens zweier muskelbezogener Signale mit verschiedenen Messfrequenzen. Da die Erfassung der beiden Signale durch dasselbe Elektrodenpaar erfolgen kann, kann der apparative Aufwand verringert werden, und es kann sichergestellt werden, dass die beiden Signale genau am selben Ort gemessen werden.

Obwohl das Messen von zwei Signalen unter Verwendung von verschiedenen Messfrequenzen gemäß dem ersten Aspekt der Erfindung von den Erfindern als besonders zuverlässig angesehen wird, kann unter Umständen die Messung von nur einem Signal, also mit nur einer Messfrequenz, und anschließender Auswertung der Phase des gemessenen Signals ausreichend sein.

Hierbei ist es bevorzugt, dass die erste Messfrequenz wesentlich größer als 60 kHz ist und / oder die Auswerteeinheit dazu eingerichtet ist, eine Phase des ersten Signals mit einer, insbesondere frequenzabhängigen, Referenzphase zu vergleichen, um eine erste Phasenveränderung wenigstens qualitativ zu ermitteln.

Hierdurch kann die Unterscheidung zwischen echten muskelbezogenen Signalen und Störsignalen mit nur einer Messung mit ausreichender Zuverlässigkeit erfolgen.

In einem Verfahren zur Steuerung oder Regelung einer Orthese oder Prothese durch ein erfindungsgemäßes Orthesen- oder Prothesen-System werden ein erstes und ein zweites muskelbezogenes Signal durch das wenigstens eine Elektrodenpaar erfasst und deren Phase durch die wenigstens eine Auswerteeinheit ausgewertet, wobei eine Phase des ersten Signals mit einer, insbesondere frequenzabhängigen, Referenzphase verglichen wird, um eine erste Phasenveränderung zu ermitteln; eine Phase des zweiten Signals mit der, insbesondere frequenzabhängigen, Referenzphase verglichen wird, um eine zweite Phasenveränderung zu ermitteln; und die erste Phasenveränderung und die zweite Phasenveränderung ausgewertet, insbesondere miteinander verglichen werden. Außerdem werden der wenigstens eine Aktor in Abhängigkeit des Ergebnisses der Auswertung der Phasen des wenigstens ersten und zweiten muskelbezogenen Signals gesteuert und die Orthese oder Prothese durch den wenigstens einen Aktor bewegt.

Durch das Verfahren kann eine schnelle und einfache Signalverarbeitung für ein erfindungsgemäßes Orthesen- oder Prothesen-System bereitgestellt werden.

Neben der klassischen Orthetik und Prothetik kann das hier vorgestellte Verfahren und System auch bei der die Steuerung von Informationstechnologie, wie z. B. PC, Spielekonsole o.ä. Anwendung finden, um dem Nutzer deren Bedienung zu erleichtern/ermöglichen. Die vom menschlichen Muskel gewonnenen Signale können auch, in Verbindung mit einem mechatronischen Gerät, zur elektromechanischen Verstärkung der Muskulatur verwendet werden.

Weitere Merkmale, Vorteile und Anwendungsmöglichkeiten der Erfindung ergeben sich aus der nachfolgenden Beschreibung im Zusammenhang mit der jeweiligen Figur. Dabei zeigen:
- Fig. 1: einen Teil eines Orthesen- oder Prothesen-Systems in schematischer Darstellung nach einer Ausführungsform;
- Fig. 2: den Verlauf des Betrages eines Bioimpedanzsignals bei verschiedenen Messfrequenzen;
- Fig. 3: den Verlauf der Phase eines Bioimpedanzsignals bei verschiedenen Messfrequenzen;
- Fig. 4: den Verlauf des Betrages eines Bioimpedanzsignals bei verschiedenen Messfrequenzen, und
- Fig. 5: den Verlauf der Phase eines Bioimpedanzsignals bei verschiedenen Messfrequenzen.

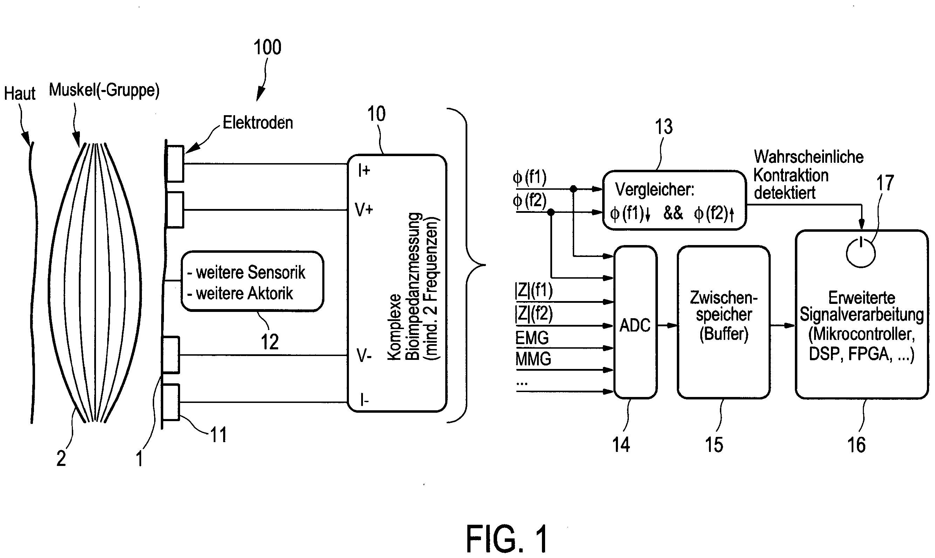

**Fig. 1** zeigt einen Teil eines Orthesen- oder Prothesen-Systems 100 nach einem Ausführungsbeispiel der vorliegenden Erfindung. Links in Fig. 1 ist ein Arm angedeutet mit Haut 1 und Muskel 2. Obwohl das erfindungsgemäße System vorwiegend in Bezug auf einen Arm beschrieben wird, ist es selbstverständlich, dass das Orthesen- oder Prothesen- System auch mit anderen Körperpartien benutzt werden kann. Auch versteht es sich, dass der hier beschriebene Muskel 2 stellvertretend für Muskeln oder Muskelgruppen steht.

Ein Satz von Elektroden 11 des Orthesen- oder Prothesen-Systems 100 kontaktieren die Haut 1, wobei der Elektrodensatz in dem gezeigten Beispiel aus vier Elektroden 11 besteht, nämlich jeweils zwei Elektroden, die der Stromeinprägung dienen, und zwei Elektronen zur Spannungsmessung. Aus der gemessenen Spannung und dem gemessenen oder im Voraus bekannten Strom kann die Bioimpedanz in an sich bekannter Weise ermittelt werden.

Die Orthese oder Prothese selbst ist nicht in Fig. 1 gezeigt. Gegebenenfalls kann, wie in Fig. 1 gezeigt, weitere Sensorik und/oder weitere Aktorik 12 vorhanden sein und gegebenenfalls die Haut 1 kontaktieren.

Die Elektroden 11 sind mit einer Auswerteeinheit 10 verbunden, wo die Strom- und Spannungssignale weiterverarbeitet und/oder gespeichert werden können. Der rechte Teil der Fig. 1 illustriert schematisch die weitere Verarbeitung der Strom- und Spannungssignale bzw. die weitere Verarbeitung von aus den Strom- und Spannungssignalen abgeleiteten Signalen. Diese Funktionen können entweder in der Auswerteeinheit 10 oder in einer daran gekoppelten, nicht dargestellten Auswerteeinheit implementiert sein.

Wie nachfolgend noch näher erläutert wird, können insbesondere die Phase ϕ und der Betrag |Z| der Bioimpedanz Z ermittelt und weiterverarbeitet werden. Optional können, wie in Fig. 1 angedeutet, auch weitere Messgrößen, wie ein Elektromyographie (EMG) und/oder Mechanomyographie-Signal (MMG), ermittelt und gegebenenfalls weiterverarbeitet werden.

Die Erfinder haben erkannt, dass sich sowohl der Betrag |Z| als auch die Phase ϕ der Bioimpedanz bei einer Muskelkontraktion ändert. Dabei haben sie festgestellt, dass die Phasenänderung in Abhängigkeit von der Messfrequenz eine Charakteristik aufweist, die für die Unterscheidung zwischen "echten" muskelbezogenen Signalen und Störsignalen benutzt werden kann. Dabei wird unter dem Begriff "echte" muskelbezogene Signale vorzugsweise verstanden, dass es sich um Signale handelt, die auf eine tatsächlich auftretende Muskelkontraktion o.Ä. zurückgeführt werden können. Im Unterschied dazu sind Störsignale vorzugsweise solche Signale, die zumindest teilweise, vorzugsweise vorwiegend, und weiter vorzugsweise im Wesentlichen ausschließlich, auf Störeinflüsse zurückzuführen sind. Ein solcher Störeinfluss kann beispielsweise ein unzureichender Kontakt zwischen einer Elektrode 11 und der Haut 1 sein.

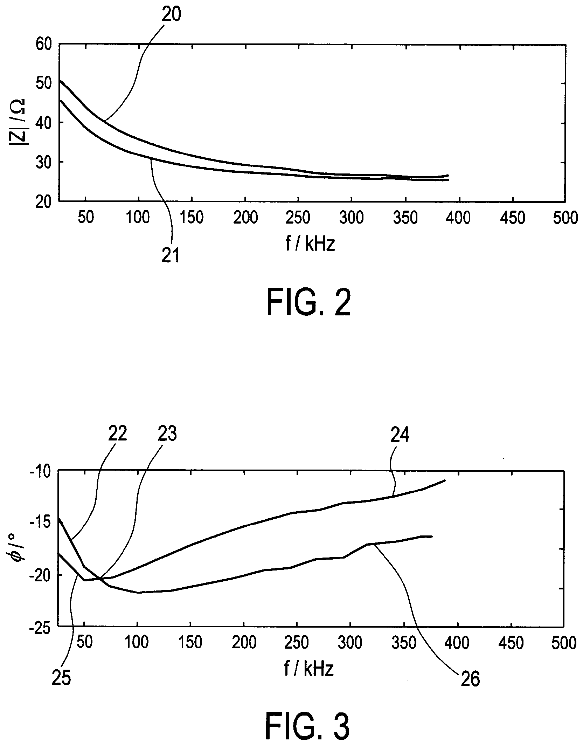

**Fig. 2** zeigt beispielhaft den Frequenzgang des Betrages |Z| der Bioimpedanz in Abhängigkeit von der verwendeten Messfrequenz f für einen Körperteil. Dabei zeigt die obere Kurve 20 den Verlauf des Betrags |Z| der Bioimpedanz in einem entspannten Zustand des Muskels und die untere Kurve 21 den Verlauf des Betrages |Z| der Bioimpedanz bei einer Muskelkontraktion. Wie Fig. 2 deutlich zeigt, verringert sich der Betrag |Z| der Bioimpedanz bei einer Muskelkontraktion in dem gesamten dargestellten Frequenzbereich.

Die in **Fig. 3** dargestellte Phase ϕ der Bioimpedanz weist einen anderen Verlauf in dem gezeigten Frequenzbereich auf. Die Kurve, die einem entspannten Muskelzustand entspricht, ist in einem linken Bereich mit dem Bezugszeichen 22 versehen und in einem rechten Bereich mit dem Bezugszeichen 26. Die Kurvenabschnitte 25 und 24 stellen die Phase der Bioimpedanz bei einer Muskelkontraktion dar. Wie in Fig. 3 deutlich zu sehen ist, schneidet die Kurve 22, 26 bei einem Punkt 23 die Kurve 24, 25. Dies bedeutet, dass sich die Phase bei einer Messfrequenz, die dem Schnittpunkt 23 entspricht (ca. 60 kHz), durch eine Muskelkontraktion gegenüber dem entspannten Zustand nicht ändert. Bei niedrigeren Messfrequenzen (links vom Schnittpunkt 23) verringert sich die Phase ϕ der Bioimpedanz aufgrund einer Muskelkontraktion. Dagegen vergrößert sich die Phase ϕ bei höheren Messfrequenzen (rechts des Schnittpunkts 23) durch eine Muskelkontraktion.

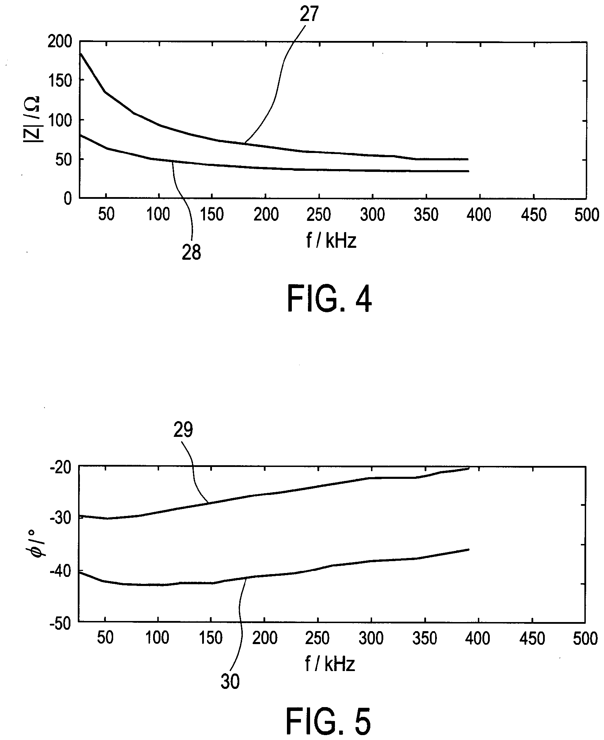

Zu der Fig. 3 (und entsprechend auch zu der im Folgenden erläuterten Fig. 5) ist anzumerken, dass ein Verringern der Phase hier bedeutet, dass die Phase "noch negativer" wird. Der Betrag der Phase vergrößert sich also bei dem in Fig. 3 veranschaulichten Verringern der Phase.

Der in Fig. 3 dargestellte Frequenzgang der Phase ϕ der Bioimpedanz kann nach Ausführungsbeispielen der vorliegenden Erfindung ausgewertet werden, um eine Muskelkontraktion zu erkennen und insbesondere, um eine solche von einem Störsignal zu unterscheiden. Die Steuereinheit, die den Aktor ansteuert, um die Orthese- oder Prothese zu bewegen, kann dann in Abhängigkeit von einem Ergebnis der mit der Auswerteeinheit 10 durchgeführten Auswertung betrieben werden.

Hinsichtlich der Messung der Bioimpedanz und der Auswertung der Phase der Bioimpedanz gibt es mehrere Varianten, die hier kurz beschrieben werden. Im Folgenden wird mehrmals auf den Begriff "Schnittfrequenz", abgekürzt fs, Bezug genommen. Diese Schnittfrequenz entspricht der Messfrequenz beim Schnittpunkt 23, wobei diese unter Umständen für verschiedene Muskeln bzw. bei verschiedenen Personen unterschiedlich sein könnte. Falls nötig, könnte diese Schnittfrequenz von Fall zu Fall empirisch bestimmt werden.

### Variante 1:

Es werden die Bioimpedanz bzw. die Phase ϕ der Bioimpedanz bei zwei verschiedenen Messfrequenzen f1 und f2 gemessen, wobei f1 kleiner, insbesondere wesentlich kleiner als fs ist, und wobei f2 größer, insbesondere wesentlich größer als fs ist. Aus diesen Messungen ergeben sich die Phasen ϕ1 und ϕ2 für die Messfrequenzen f1 bzw. f2. Die Phasen ϕ1 und ϕ2 werden dann in der Auswerteeinheit 10 mit einer Referenzphase ϕR verglichen. Die Referenzphase ϕR kann im Voraus in der Auswerteeinheit 10 abgespeichert worden sein. Die Referenzphase ϕR kann insbesondere die Phase ϕ der Bioimpedanz im entspannten Muskelzustand sein, also wie durch Kurve 22, 26 dargestellt. Obwohl es sinnvoll sein kann, die Referenzphase ϕR für viele verschiedene Frequenzen zu ermitteln und abzuspeichern, wäre es auch möglich, die Referenzphase ϕR nur für die zwei zu verwendenden Messfrequenzen f1 und F zu ermitteln und abzuspeichern.

In der Auswerteeinheit 10, insbesondere durch einen Vergleicher 13 (Fig. 1), werden nun die Phasen ϕ1 und ϕ2 mit entsprechenden Referenzphasen ϕR1 und ϕR2 verglichen. Durch einen solchen Vergleich kann eine Phasenveränderung oder Phasendifferenz ϕD1 und ϕD2 ermittelt werden. Diese Phasenveränderung bzw. Phasendifferenz entspricht also der durch die Muskelkontraktion hervorgerufene Veränderung der Phasen bei den jeweiligen Messfrequenzen. Stellt der Vergleicher 13 für die beiden Messfrequenzen Phasenveränderungen in unterschiedliche Richtungen fest (bzw. Phasendifferenzen mit unterschiedlichen Vorzeichen), wird das durch die Elektroden 11 gemessene Signal als echtes Signal einer Muskelkontraktion gewertet.

Stellt der Vergleicher 13 hingegen Phasenveränderungen in die gleiche Richtung bzw. Phasendifferenzen mit gleichem Vorzeichen fest, so wird das durch die Elektroden 11 gemessene Signal als Störsignal gewertet, oder zumindest nicht als zu einer Muskelkontraktion gehörend.

Falls keine Phasenveränderung oder -differenz festzustellen ist, oder wenn die Phasenveränderung oder-differenz einen Mindestwert nicht überschritten hat, wird das Signal nicht als zu einer Muskelkontraktion gehörend gewertet, d.h. in diesem Fall wird die Messung vorzugsweise so interpretiert, dass der Muskel im entspannten Zustand ist.

### Variante 2:

Die zweite Variante unterscheidet sich von der ersten insbesondere dadurch, dass eine der Messfrequenzen f1, f2 in der Nähe der Schnittfrequenz fs liegt, insbesondere im Wesentlichen der Schnittfrequenz gleicht. Die Auswertung wird entsprechend angepasst. Das heißt insbesondere, dass ein durch die Elektroden 11 detektiertes Signal als echtes muskelbezogenes Signal gewertet wird, wenn für die Messfrequenz, die sich (wesentlich) von der Schnittfrequenz fs unterscheidet, eine Phasenveränderung oder Phasendifferenz festgestellt wird, für die andere Messfrequenz, die nahe oder gleich der Schnittfrequenz fs ist, hingegen keine oder nur eine sehr geringe Phasenveränderung oder-differenz festgestellt wird.

Das Signal wird hingegen als nicht zu einer Muskelkontraktion gehörend gewertet, wenn sich für beide Messfrequenzen keine bzw. nur eine sehr geringe Veränderung der Phase ergibt, oder wenn sich für beide Messfrequenzen eine Phasenveränderung in die gleiche Richtung ergibt.

Bei der ersten und zweiten Variante wäre es möglich, die Auswertung nur qualitativ durchzuführen, also nur zu ermitteln, in welche Richtungen die Phasen sich verändern (bzw. ob überhaupt eine Phasenveränderung vorliegt), ohne die Größe der Veränderung (weiter) zu berücksichtigen. Eine solche qualitative Auswertung würde ausreichen, um ein echtes muskelbezogenes Signal von einem Störsignal zu unterscheiden.

### Variante 3:

Diese Variante ist der zweiten Variante ähnlich. Bei der dritten Variante werden die Messfrequenzen f1 und f2 so gewählt, dass entweder beide größer oder beide kleiner als die Schnittfrequenz fs sind. Dabei sollte aber eine der Frequenzen deutlich näher an der Schnittfrequenz liegen als die andere. Nach dieser Variante wäre es aber nötig, die Phasenveränderung bzw. Phasendifferenz nicht nur qualitativ (Phasenveränderung in gleiche oder unterschiedliche Richtungen, Phasendifferenzen mit gleichen oder unterschiedlichen Vorzeichen, keine wesentliche Phasenveränderung), sondern auch quantitativ auszuwerten, weil zu erwarten wäre, dass bei einer Muskelkontraktion die Phasen bei den verschiedenen Messfrequenzen sich in die gleiche Richtung verändern würden, dass die Größe der Veränderungen aber unterschiedlich wäre.

### Variante 4:

Nach dieser Variante reicht es aus, nur eine Phase, also nur bei einer Messfrequenz, zu ermitteln und auszuwerten. Die Messfrequenz liegt dabei vorzugsweise oberhalb der Schnittfrequenz fs und ist insbesondere wesentlich höher als die Schnittfrequenz fs, also in einem Frequenzbereich, für den in der Fig. 3 die Kurve 24 (Muskelkontraktion) wesentlich höher liegt als die Kurve 26 (entspannter Zustand). Es wird wiederum die Phasenveränderung relativ zu einer Referenzphase ausgewertet, beispielsweise durch das Bilden einer Phasendifferenz. Dies kann wiederum in dem Vergleicher 13 durchgeführt werden. Stellt der Vergleicher 13 fest, dass die aus der Messung ermittelte Phase größer ist als die Referenzphase, wird das Signal als zu einer Muskelkontraktion gehörend gewertet. Anderenfalls wird das Signal entweder als Störsignal (die aus der Messung ermittelte Phase ist kleiner als die Referenzphase) oder als zu einem entspannten Muskel (keine Phasenveränderung) gehörend gewertet.

### Weitere Erläuterungen

Es wurde bereits erwähnt, dass Orthesen- oder Prothesen-Systeme nach Ausführungsformen der Erfindung echte muskelbezogene Signale, also insbesondere auf eine Muskelkontraktion zurückzuführende Signale, mit größerer Zuverlässigkeit erkennen können und insbesondere von Störsignalen unterscheiden können, als dies nach Ansätzen im Stand der Technik möglich war. In diesem Zusammenhang ist es zweckmäßig zu erläutern, welchen Verlauf der Betrag |Z| der Bioimpedanz bzw. die Phase ϕ der Bioimpedanz eines möglichen Störsignals aufweist. In diesem Zusammenhang wird auf die Figuren 4 und 5 Bezug genommen.

**Fig. 4** zeigt den frequenzabhängigen Verlauf des Betrages |Z| der Bioimpedanz für einen gegebenen Muskelzustand. Hierbei zeigt die untere Kurve 28 den Verlauf bei einem guten Elektrode-Haut-Kontakt der negativen Strom-Elektrode und Kurve 27 den Verlauf bei einem schlechten Elektrode-Haut-Kontakt. Eine betragsmäßig größere Bioimpedanz (obere Kurve 27) entspricht also einem schlechteren Kontakt als eine betragsmäßig kleinere Bioimpedanz (untere Kurve 28). Eine Auswertung der Signale, die sich allein auf den Betrag |Z| der Bioimpedanz stützt, könnte aber unter Umständen unzuverlässige Ergebnisse liefern. So kann beispielsweise ein Zunehmen des Betrages der Bioimpedanz entweder bedeuteten, dass sich der Muskel gegenüber einem Vergleichszustand (mehr) entspannt hat, oder dass sich der Elektrode-Haut-Kontakt verschlechtert hat.

**Fig. 5** zeigt den Frequenzgang der Phase der Bioimpedanz im Falle eines guten Elektrode- Haut-Kontaktes (obere Kurve 29) und im Falle eines schlechten Elektrode-Haut-Kontaktes (untere Kurve 30). Der in Fig. 5 gezeigte Frequenzgang der Phase weist nicht den in Fig. 3 gezeigten charakteristischen Frequenzgang für echte muskelbezogene Signale auf. Insbesondere schneiden sich die Kurven 29 und 30 nicht. Die Unterschiede zwischen einem echten muskelbezogenen Signal (Fig. 3) und einem Störsignal, wie beispielsweise einem schlechten Elektrode-Haut-Kontakt (Fig. 5), werden besonders deutlich, wenn man eine Messung und Auswertung gemäß der oben beschriebenen ersten Variante durchführt. Bei den zwei Messungen mit den zwei Messfrequenzen f1 und f2 (mit f1 < fs < f2) liefert die zuvor beschriebene Auswertung für ein echtes muskelbezogenes Signal eine Phasenveränderung in verschiedene Richtungen. Für ein Störsignal, wie z.B. eine Verschlechterung des Elektrode-Haut-Kontaktes, liefert sie dagegen eine Phasenveränderung in die gleiche Richtung. Aufgrund dieser Tatsache kann die Auswerteeinheit 10 zwischen einem echten muskelbezogenen Signal und einem Störsignal unterscheiden.

Ähnliches gilt für die weiteren oben beschriebenen Varianten.

Nach Ausführungsformen der Erfindung können zwei oder mehr Messungen bei zwei oder mehr unterschiedlichen Frequenzen mittels des gleichen Satzes von Elektroden 11 (unter Verwendung von mehr als einem Messkanal) durchgeführt werden. Die Messungen könnten quasi-simultan erfolgen, d.h. mit für einen Anwender nicht merklichen, kurzen Abständen. Alternativ könnten die Messungen bei den zwei oder mehr unterschiedlichen Frequenzen mittels verschiedener Sätze von Elektroden durchgeführt werden.

Wie in Fig. 1 gezeigt, weist die Auswerteeinheit 10 gemäß dem gezeigten Ausführungsbeispiel einen A/D-Wandler (ADC) 14 auf, der die ermittelte Phase der Bioimpedanz und gegebenenfalls auch den Betrag der Bioimpedanz und/oder andere gemessene oder daraus abgeleitete Größen in ein digitales Format umwandelt. Die von dem A/D-Wandler ausgegebenen digitalen Daten können in einem Zwischenspeicher (Buffer) 15 zwischengespeichert werden und gegebenenfalls durch eine erweiterte Signalverarbeitungseinheit 16 weiterverarbeitet werden. Insbesondere kann ein solches Weiterverarbeiten das Steuern eines Aktors zur Bewegung der Orthese oder Prothese aufweisen.

Fig. 1 weist noch auf einen weiteren Vorteil von Ausführungsformen der Erfindung hin. Ausführungsformen der Erfindung sehen vor, dass zusätzliche Messverfahren und/oder Signalverarbeitungskomponenten erst eingeschaltet werden, wenn eine Muskelkontraktion mittels der zuvor beschriebenen Verfahren, insbesondere durch den Vergleicher 13, detektiert wurde. Dies ist in Fig. 1 durch einen Pfeil angedeutet, der von dem Vergleicher 13 ausgeht und auf das Symbol eines Ein/Aus-Schalters 17 der Einheit 16 führt. So können Prozesse bzw. Komponenten mit unter Umständen vergleichsweise hohem Energiebedarf energiesparender ausgeführt bzw. betrieben werden, was den Energiebedarf des gesamten Systems verringern kann. Dadurch kann eventuell Batteriekapazität der Orthese oder Prothese bzw. des Orthesen- oder Prothesen-Systems eingespart werden, was auch zu Vergünstigungen bei der Herstellung führen kann.

Nach einer weiteren Ausgestaltung der Erfindung ist es möglich, mehrere Elektroden(sätze) 11 in einem Elektrodenarray anzuordnen. Dadurch ist eine hohe geometrische Auflösung realisierbar.

Vorteile (oder weitere Vorteile) von zumindest manchen Ausgestaltungen der Erfindung sind:
- Kostengünstiges System, beispielsweise dadurch, dass kein (signifikant) höherer Schaltungsaufwand im Vergleich zu bereits bekannten Systemen entsteht
- (Signifikante) Erhöhung der Messsicherheit von Muskelkontraktionen und bessere Erkennung des Nutzerwunsches nach einer Prothesenbewegung
- Geringe Anzahl von Elektroden notwendig

Mögliche Erweiterungen / Varianten:
- Wie die anderen Komponenten auch, kann der Vergleicher sowohl digital, als auch analog realisiert werden.
- Zusätzlich kann das Verfahren um die Information der Bioimpedanz-Betragsänderungen bei Muskelkontraktion erweitert werden.
- Das anisotrope Impedanzverhalten von Muskelgewebe kann ausgenutzt werden, indem mehrere (quasi-)simultane Messungen an der gleichen Muskelgruppe in unterschiedlichen geometrischen Elektroden-Anordnungen durchgeführt werden.
- Messung des Zustands der Elektrode-Haut-Übergänge. Aus dieser Information kann auch geschlossen werden, wie zuverlässig die zusätzliche Messung des EMG-Signals ist.
- Mittels der Bioimpedanzmessung kann auch die arterielle Pulswelle detektiert werden. Aus dieser lassen sich Informationen wie Herzschlagfrequenz, Pulswellengeschwindigkeit, Augmentationsindex, Blutdruck und Atemfrequenz ableiten.
- Der entstehende Informationsgewinn durch das Messen bei mindestens 2 Frequenzpunkten liefert zusätzliche Informationen über die Kontraktionsstärke.

Mögliche Einsatzgebiete der Erfindung sind unter anderem Prothetik, Orthetik, Mensch-Maschine-Schnittstelle zum Ansteuern von Computern, Maschinensteuerung und Ansteuerung von Exoskeletten.

### Bezugszeichenliste

- 1: Haut
- 2: Muskel
- 10: Auswerteeinheit
- 11: Elektrodenpaar
- 12: weitere Sensorik/Aktorik
- 13: Vergleicher
- 14: A/D-Wandler
- 15: Zwischenspeicher
- 16: Signalverarbeitungseinheit
- 17: Schalter
- 20,21,27,28: Messkurven (Betrag der Bioimpedanz)
- 22,24-26,29,30: Messkurven (Phase der Bioimpedanz)
- 100: Orthesen- oder Prothesen-System

## Patentansprüche

1. Orthesen- oder Prothesen-System (100), aufweisend
- wenigstens eine Orthese oder Prothese,
- wenigstens ein Paar von Elektroden, als Elektrodenpaar (11), welche zur Kontaktierung des Körpers (1) des Nutzers der jeweiligen Orthese oder Prothese vorgesehen sind, zur Erfassung muskelbezogener Signale,
- wenigstens eine Auswerteeinheit (10) für von dem wenigstens einen Elektrodenpaar (11) erfasste muskelbezogene Signale,
- wenigstens einen Aktor (12) zur Bewegung der jeweiligen wenigstens einen Orthese oder Prothese und
- wenigstens eine Steuereinheit zur Steuerung des wenigstens einen Aktors (12), wobei das wenigstens eine Elektrodenpaar (11) dazu eingerichtet ist, wenigstens ein erstes muskelbezogenes Signal unter Verwendung einer ersten Messfrequenz und ein zweites muskelbezogenes Signal unter Verwendung einer zweiten Messfrequenz zu erfassen und die wenigstens eine Auswerteeinheit (10) dazu eingerichtet ist, eine Phase des ersten Signals und eine Phase des zweiten Signals auszuwerten **dadurch gekennzeichnet, dass** die Auswerteeinheit (10) dazu eingerichtet ist:
- eine Phase des ersten Signals mit einer, insbesondere frequenzabhängigen, Referenzphase zu vergleichen, um eine erste Phasenveränderung zu ermitteln;
- eine Phase des zweiten Signals mit der, insbesondere frequenzabhängigen, Referenzphase zu vergleichen, um eine zweite Phasenveränderung zu ermitteln; und
- die erste Phasenveränderung und die zweite Phasenveränderung auszuwerten, insbesondere miteinander zu vergleichen.

2. Orthesen- oder Prothesen-System (100) nach Anspruch 1, **dadurch gekennzeichnet, dass** die Auswerteeinheit (10) dazu eingerichtet ist, einen Phasenverlauf des ersten und zweiten Signals auszuwerten.

3. Orthesen- oder Prothesen-System (100) nach einem der voranstehenden Ansprüche, **dadurch gekennzeichnet, dass** die Referenzphase eine, insbesondere zuvor bestimmte, Phase (22,26) ist, die im Wesentlichen einer Messung in einem entspannten Zustand eines Muskels (2) entspricht.

4. Orthesen- oder Prothesen-System (100) nach einem der voranstehenden Ansprüche, **dadurch gekennzeichnet, dass** die Auswerteeinheit (10) dazu eingerichtet ist zu ermitteln, ob die erste Phasenveränderung eine Phasenveränderung in die gleiche Richtung wie die zweite Phasenveränderung ist, oder in eine entgegengesetzte Richtung.

5. Orthesen- oder Prothesen-System (100) nach Anspruch 4, **dadurch gekennzeichnet, dass** die Auswerteeinheit (10) dazu eingerichtet ist, das erste und zweite Signal als zu einer Muskelkontraktion gehörend zu werten, wenn die erste und zweite Phasenveränderung Phasenveränderungen in entgegengesetzte Richtungen sind.

6. Orthesen- oder Prothesen-System (100) nach Anspruch 4 oder 5, **dadurch gekennzeichnet, dass** die Auswerteeinheit (10) dazu eingerichtet ist, das erste und zweite Signal als zu einer Störung gehörend zu werten, wenn die erste und zweite Phasenveränderung Phasenveränderungen in die gleiche Richtung sind.

7. Orthesen- oder Prothesen-System (100) nach einem der vorhergehenden Ansprüche, **dadurch gekennzeichnet, dass** die erste Messfrequenz mehr als ca. 60kHz beträgt und die zweite Messfrequenz weniger als ca. 60kHz beträgt.

8. Orthesen- oder Prothesen-System (100) nach einem der vorhergehenden Ansprüche, **dadurch gekennzeichnet, dass** die erste Messfrequenz wesentlich größer als 60kHz ist und / oder die zweite Messfrequenz wesentlich kleiner als 60kHz ist.

9. Orthesen- oder Prothesen-System (100) nach einem der vorhergehenden Ansprüche, **dadurch gekennzeichnet, dass** das wenigstens erste und zweite muskelbezogene Signal ein Bioimpedanz-Signal ist.

10. Orthesen- oder Prothesen-System (100) nach einem der vorhergehenden Ansprüche, **dadurch gekennzeichnet, dass** das wenigstens eine Elektrodenpaar (11) weiterhin dazu eingerichtet ist, dem Nutzer der Orthese oder Prothese elektrische Stimulationssignale zuzuführen.

11. Orthesen- oder Prothesen-System (100)nach Anspruch 10, **dadurch gekennzeichnet, dass** die elektrischen Stimulationssignale dazu eingerichtet sind, dem Nutzer der Orthese oder Prothese ein Feedback über die Bewegungen und / oder den Zustand der Orthese oder Prothese zu geben.

## Claims

1. An orthosis or prosthesis system (100), comprising
- at least one orthosis or prosthesis,
- at least one pair of electrodes as an electrode pair (11), provided for contacting the body (1) of the user of the respective orthosis or prosthesis for the purpos of detecting muscle-related signals,
- at least one evaluation unit (10) for muscle-related signals detected by the at least one electrode pair (11),
- at least one actuator (12) for moving the respective at least one orthosis or prosthesis, and
- at least one control unit for controlling the at least one actuator (12), wherein the at least one electrode pair (11) is configured to detect at least a first muscle-related signal using a first measurement frequency and a second muscle-related signal using a second measurement frequency and the at least one evaluation unit (10) is configured to evaluate a phase of the first signal and a phase of the second signal **characterized in that** the evaluation unit (10) is configured:
- to compare a phase of the first signal to a reference phase, in particular to a frequency-dependent reference phase, in order to ascertain a first phase change;
- to compare a phase of the second signal to the reference phase, in particular to the frequency-dependent reference phase, in order to ascertain a second phase change; and
- to evaluate the first phase change and the second phase change, in particular to compare these to one another.

2. The orthosis or prosthesis system (100) as claimed in claim 1, **characterized in that** the evaluation unit (10) is configured to evaluate a phase response of the first and second signal.

3. The orthosis or prosthesis system (100) as claimed in any one of the preceding claims , **characterized in that** the reference phase is a phase (22, 26), in particular a phase determined in advance, which substantially corresponds to a measurement in a relaxed state of a muscle.

4. The orthosis or prosthesis system (100) as claimed in any of the preceding claims, **characterized in that** the evaluation unit (10) is configured to ascertain whether the first phase change is a phase change in the same direction as the second phase change or in an opposite direction thereto.

5. The orthosis or prosthesis system (100) as claimed in claim 4, **characterized in that** the evaluation unit (10) is configured to assess the first and second signal as belonging to a muscle contraction if the first and second phase change are phase changes in opposite directions.

6. The orthosis or prosthesis system (100) as claimed in claim 4 or 5, **characterized in that** the evaluation unit (10) is configured to assess the first and second signal as belonging to a disturbance if the first and second phase change are phase changes in the same direction.

7. The orthosis or prosthesis system (100) as claimed in any one of the preceding claims, **characterized in that** the first measurement frequency is more than approximately 60 kHz and the second measurement frequency is less than approximately 60 kHz.

8. The orthosis or prosthesis system as claimed in any one of the preceding claims, **characterized in that** the first measurement frequency is substantially greater than 60 kHz and/or the second measurement frequency is substantially less than 60 kHz.

9. The orthosis or prosthesis system (100) as claimed in any one of the preceding claims, **characterized in that** the at least first and second muscle-related signal is a bioimpedance signal.

10. The orthosis or prosthesis system (100) as claimed in any one of the preceding claims, **characterized in that** the at least one electrode pair (11) is further configured to supply electrical stimulation signals to the user of the orthosis or prosthesis.

11. The orthosis or prosthesis system (100) as claimed in claim 10, **characterized in that** the electrical stimulation signals are configured to provide the user of the orthosis or prosthesis with feedback about the movements and/or the state of the orthosis or prosthesis.

## Revendications

1. Système orthétique ou prothétique (100), comprenant
- au moins une orthèse ou une prothèse,
- au moins une paire d'électrodes sous forme de paire d'électrodes (11) prévues pour venir en contact avec le corps (1) de l'utilisateur de l'orthèse ou de la prothèse respective, afin de détecter des signaux relatifs aux muscles,
- au moins une unité d'évaluation (10) pour les signaux relatifs aux muscles détectés par ladite au moins une paire d'électrodes (11),
- au moins un actionneur (12) pour déplacer ladite au moins une orthèse ou prothèse respective, et
- au moins une unité de commande pour commander ledit au moins un actionneur (12),
dans lequel
ladite au moins une paire d'électrodes (11) est conçue pour détecter au moins un premier signal relatif aux muscles en utilisant une première fréquence de mesure, et un deuxième signal relatif aux muscles en utilisant une deuxième fréquence de mesure, et
ladite au moins une unité d'évaluation (10) est conçue pour évaluer une phase du premier signal et une phase du deuxième signal,
**caractérisé en ce que**
l'unité d'évaluation (10) est conçue pour
- comparer une phase du premier signal avec une phase de référence, en particulier dépendante de la fréquence, afin de déterminer un premier changement de phase ;
- comparer une phase du deuxième signal avec la phase de référence, en particulier dépendante de la fréquence, afin de déterminer un deuxième changement de phase ; et
- évaluer le premier changement de phase et le deuxième changement de phase, en particulier en les comparant l'un à l'autre.

2. Système orthétique ou prothétique (100) selon la revendication 1,
**caractérisé en ce que**
l'unité d'évaluation (10) est conçue pour évaluer une évolution de phase des premier et deuxième signaux.

3. Système orthétique ou prothétique (100) selon l'une des revendications précédentes,
**caractérisé en ce que**
la phase de référence est une phase (22, 26), en particulier déterminée au préalable, correspondant sensiblement à une mesure dans un état détendu d'un muscle (2).

4. Système orthétique ou prothétique (100) selon l'une des revendications précédentes,
**caractérisé en ce que**
l'unité d'évaluation (10) est conçue pour déterminer si le premier changement de phase est un changement de phase dans la même direction que le deuxième changement de phase, ou dans une direction opposée.

5. Système orthétique ou prothétique (100) selon la revendication 4,
**caractérisé en ce que**
l'unité d'évaluation (10) est conçue pour évaluer les premier et deuxième signaux comme appartenant à une contraction musculaire, si les premier et deuxième changements de phase sont des changements de phase dans des directions opposées.

6. Système orthétique ou prothétique (100) selon la revendication 4 ou 5,
**caractérisé en ce que**
l'unité d'évaluation (10) est conçue pour évaluer les premier et deuxième signaux comme appartenant à un défaut, si les premier et deuxième changements de phase sont des changements de phase dans la même direction.

7. Système orthétique ou prothétique (100) selon l'une des revendications précédentes,
**caractérisé en ce que**
la première fréquence de mesure est supérieure à environ 60 kHz et la deuxième fréquence de mesure est inférieure à environ 60 kHz.

8. Système orthétique ou prothétique (100) selon l'une des revendications précédentes,
**caractérisé en ce que**
la première fréquence de mesure est sensiblement supérieure à 60 kHz et/ou la deuxième fréquence de mesure est sensiblement inférieure à 60 kHz.

9. Système orthétique ou prothétique (100) selon l'une des revendications précédentes,
**caractérisé en ce que**
au moins le premier et le deuxième signal relatif au muscle est un signal de bio-impédance.

10. Système orthétique ou prothétique (100) selon l'une des revendications précédentes,
**caractérisé en ce que**
ladite au moins une paire d'électrodes (11) est en outre conçue pour délivrer des signaux de stimulation électriques à l'utilisateur de l'orthèse ou de la prothèse.

11. Système orthétique ou prothétique (100) selon la revendication 10,
**caractérisé en ce que**
les signaux de stimulation électriques sont conçus pour fournir un retour d'information à l'utilisateur de l'orthèse ou de la prothèse concernant les mouvements et/ou la condition de l'orthèse ou de la prothèse.
